# EUROPEAN PATENT SPECIFICATION

(11) **EP 3 328 452 B1**
(45) Date of publication and mention of the grant of the patent: **09.12.2020**
(21) Application number: 16750671.6
(22) Date of filing: 28.07.2016
(51) Int. Cl.: A61L 27/10, A61L 27/30, A61L 27/50

(54) **METALLIC AND/OR CERAMIC COMPONENTS WITH AT LEAST ONE OSSEOINTEGRATIVE AND OSTEOINDUCTIVE SURFACE (MULTI)LAYER STRUCTURE**
METALL- UND/ODER KERAMIKKOMPONENTEN MIT ZUMINDEST EINER OSSEOINTEGRATIVEN UND OSTEOINDUKTIVEN OBERFLÄCHEN(MEHR)SCHICHTSTRUKTUR
COMPOSANTS MÉTALLIQUES ET/OU CÉRAMIQUES COMPORTANT AU MOINS UNE STRUCTURE MULTICOUCHE À SURFACE OSTÉOINTÉGRATIVE ET OSTÉOINDUCTIVE

(30) Priority: 28.07.2015 EP 15178661
(43) Date of publication of application: 06.06.2018
(73) Proprietor: CeramTec GmbH, 73207 Plochingen (DE)
(72) Inventor: PORPORATI, Alessandro Alan, 73207 Plochingen (DE); RASCHKE, Marita, 71229 Leonberg (DE); STREICHER,Robert, 8835 Feusisberg (CH); KUNTZ, Meinhard, 73733 Esslingen (DE); PREUSS, Roman, 73230 Kirchheim unter Teck (DE); GOTTWIK, Lukas, 73087 Bad Boll (DE)
(74) Representative: Fehrenbacher, Eckhard Anton
(86) International application number: PCT/EP2016/067973
(87) International publication number: WO 2017/017167

(56) References cited:
- WO-A1-01/72664
- ALPASLAN SENKOYLU ET AL: "Effect of Strontium Ranelate on Hydrogen Peroxide-Induced Apoptosis of CRL-11372 Cells", BIOCHEMICAL GENETICS, KLUWER ACADEMIC PUBLISHERS-PLENUM PUBLISHERS, NE, vol. 46, no. 3-4, 26 January 2008 (2008-01-26), pages 197-205, XP019569968, ISSN: 1573-4927
- MARIE P J ET AL: "MECHANISMS OF ACTION AND THERAPEUTIC POTENTIAL OF STRONTIUM IN BONE", CALCIFIED TISSUE INTERNATI, NEW YORK, NY, US, vol. 69, no. 3, 1 January 2001 (2001-01-01), pages 121-129, XP008040696, ISSN: 0171-967X

## Description

The present invention relates to metallic and / or ceramic components, in particular in the field of medical technology, having improved osseointegrative and osteoinductive properties, the metallic and / or ceramic components are suitable for a cementless fixation in the human body, for example the acetabular region of the human pelvis. The present invention also relates to a method for producing the metallic and / or ceramic components.

Nowadays metal shells with osseointegrative surface structures are necessary for a cementless fixation of ceramic inserts in the acetabular region of the human pelvis. One possibility to fix ceramic implants directly into bone is described for example in EP 1268364 A1, wherein a ceramic body is layered with a surface structure having open pores.

However, it was an object of the present invention to further enhance the osseointegrative properties, that is the ability to promote bone ingrowth, of metallic and / or ceramic components

This object is solved by an implant comprising a metallic and/or ceramic component comprising an osseointegrative and osteoinductive surface (multi)layer structure, wherein the surface (multi)layer structure comprises at least one osseointegrative and osteoinductive layer wherein the osseointegrative and osteoinductive layer comprises a ceramic base material, selected from the group consisting of alumina based ceramics and/or zirconia based ceramics, and 1-50 wt% strontium aluminate based on the total weight of the surface (multi)layer structure according to claim 1. It also enables the direct implantation of the metallic and / or ceramic component due to the addition of the porous osseointegrative and osteoinductive surface (multi)layer structure comprising strontium aluminate to the backside of a ceramic substrate.

The implant comprising the osseointegrative surface (multi)layer structure of the present invention is able to increase the bone growth rate due to the presence of strontium aluminate in the osseointegrative and osteoinductive based layer on the metallic or ceramic surface of the metallic and / or ceramic component. The surface (multi)layer structure may have also osteoinductive properties and therefore may promote the growth, maturation and activity of osteoblasts. Strontium is known as bone forming agent. Strontium-containing drugs are already used as systemic osteoblast-activating medication in various clinical settings promoting mechanical stability of the osteoporotic bone, but have not been described as an active ingredient for promoting bone ingrowth of ceramic components, in particular implants.

The term metallic and / or ceramic component as used in the present invention comprises components composed of metals only or ceramic materials only, but also components being mainly composed of ceramics and having a metallic surface for example. Preferred metallic and / or ceramic components of the present invention are ceramic components used in the field of medical technology.

The implant is, for example a cup in hip arthroplasty or a cage for the spine.

The ceramic component of the present invention comprises an osseointegrative and osteoinductive surface (multi)layer structure on its surface comprising at least one osseointegrative and osteoinductive layer. The osseointegrative surface structure is preferably a multilayer structure and comprises several osseointegrative layers, for example up to 30 layers. Preferably, the osseointegrative surface (multi)layer structure comprises from 1 to 5 osseointegrative layers.

The osseointegrative and osteoinductive surface (multi)layer structure of the present invention comprises a ceramic base material and strontium aluminate. The term strontium aluminate comprises any known strontium aluminate compound, such as the base strontium aluminate (SrAl₂O₄), monoclinic strontium aluminate (SrAl₄O₇), cubic strontium aluminate (Sr₃Al₂O₆), the hexagonal strontium aluminate (strontium hexaaluminate; SrAl₁₂O₁₉) and orthorhombic strontium aluminate (Sr₄Al₁₄O₂₅). The preferred compound is strontium hexaaluminate. The strontium aluminate is preferably present in platelet-form within the osseointegrative surface (multi)layer structure. The content of the strontium aluminate may be in the range of from 1 wt% to 50 wt%, preferably 1 wt% to 20 wt% based on the total weight of the surface (multi)layer structure.

The ceramic base material is selected from the group consisting of alumina based ceramics, zirconia based ceramics, preferably alumina based ceramics and zirconia based ceramics, more preferably a zirconia-platelet toughened alumina ceramic. The zirconia content in the zirconia-platelet toughened alumina ceramic is up to 25 wt% based on the total weight of the surface (multi)layer structure.

The osseointegrative and osteoinductive surface (multi)layer structure may exhibit a strontium gradient by means of different strontium contents in different layers of the osseointegrative surface (multi)layer structure with the aim to gradually release the residual stresses in the surface (multi)layer structure. In a preferred embodiment, the strontium content increases with the layer number, wherein the first / innermost layer has the lowest strontium content and the last / outermost layer has the largest strontium content. The outermost layer has to be seen as the layer coming in direct contact with the surrounding tissue or bone of the human body.

The amount of the strontium aluminate in the innermost layer of the respective strontium gradient structure is less than 5 wt%, preferably equal to or less than 1 wt% based on the total weight of the surface (multi)layer structure, with the proviso that the strontium content is not zero. The outermost layer comprises strontium aluminate in an amount of less than 25 wt%, preferably between 15 and 20 wt% based on the total weight of the surface (multi)layer structure.

The porosity of the osseointegrative and osteoinductive surface (multi)layer structure, that is the number of pores per unit volume, may be between 25% and 90%, preferably between 25% and 70%. The diameter of the pores of between is between 1 micron and 1000 microns, preferably between 20 microns and 600 microns. The number of pores per unit volume, their size, that is, its diameter, and its shape can advantageously be determined by the selection of a suitable pore-forming substance.

The thickness of the whole layer is between about 0.02 mm and 10 mm, preferably between 0.1 mm and 2 mm.

For the production of a ceramic component with an osseointegrative and osteoinductive surface multilayer structure, such as a highly osseointegrative and osteoinductive implant, a high content strontium alumina zirconia-based ceramic slurry can be deposited on a green substrate by using the method as described for example in EP 1268364 A1, where the substrate is formed as a green body of an inorganic material and to the substrate in the state of the green body, a suspension the same inorganic material constituting the substrate, or other material is applied. This suspension contains the layer material and additionally may contain a pore-forming substance or the pore-forming substance can also be applied separately. Only after applying the layer is a common heat treatment of substrate and layer by drying and sintering to produce a monolithic molding. The method for producing the substrate does not generally differ from those known from the prior art.

During the sintering, the green substrate achieves the full density; on the other hand the surface (multi)layer structure remains micro-porous, because of lower sintering activity caused by the high volume platelets content; and macro porous, because of pore forming or foaming agents. The diameter of the micro-pores is of between 0.05 micron to 5 micron, but preferably at submicron-level, from 0,1 micron to 1 micron, whilst the macro-pores have a diameter between 1 and 100 micron, preferably between 10 and 500 micron, with the proviso that the macro-pores are larger in diameter than the micro-pores. Moreover, it has to be understood that the micropores should look like "caves" into the material and the macropores like "valleys".

The alumina-based ceramic slurry comprises alumina, zirconia, strontium oxide and yttrium oxide. During the sintering stage, the solid reaction between alumina and strontium oxide undergoes forming the strontium aluminate, in particular the strontium hexaaluminate.

In order to achieve a macro-porous osseointegrative and osteoinductive surface (multi)layer structure the respective ceramic slurry may also comprise (organic) additives such as pore forming agents or foaming agents based on the respective method for forming a porous layer. Such additives may be present in amounts of from 0.05 to 10 wt% based on the total weight of the surface multilayer structure composition. While the pore forming agent is pyrolized during the sintering process, the foaming agent is used in foaming process both leading to a porous osseointegrative and osteoinductive layer(s) having a porosity as disclosed above.

Thus, the porosity may be enhanced with organic pore-forming agents in the sintering process or with pore-forming agents or foaming agents and foaming procedures, respectively.

As pore-forming substances, in particular organic compounds, for example, starches, cellulose or waxes, and natural and synthetic polymers, which evaporate during the thermal treatment of the substrate and the deposited thereon layer gasify to consume or burn, thereby forming the pores are used.

The ceramic slurry may comprise further additives commonly used in the field of ceramic technology, such as binder.

Alternatively, it is also possible to apply the osseointegrative layer(s) on a substrate in sintered state. Thereby, the surface (multi)layer structure may be deposited on the sintered ceramic substrate or *ad-hoc* prepared metallic substrate by vapour- or plasma-deposition processes.

The osseointegrative and osteoinductive surface (multi)layer structure may be also grown on pre-existing macro-porous surface which might be ceramic foam or metallic osseointegrative surface. Micro- and macro- porosities are produced for an improved osseointegration and osteoinduction.

The osseointegrative and osteoinductive surface (multi)layer structure may also be deposited on structured surfaces of the ceramic component. A structured surface in the sense of the present invention is for example a porous surface. The structuring of the ceramic component surface may be produced by milling processes, turning processes or a combination of both. Another method for producing a structured surface is the method of ceramic injection molding. The structuring of the metallic or ceramic component surface may be produced by for example by additive manufacturing.

Alternatively, pore forming agents which are pyrolized during the sintering or foaming agents for foaming processes can be added to the substrate material in order to produce a structured surface.

Other methods for producing structured surfaces are grinding or electro discharge machining processes.

## Claims

1. An implant comprising a metallic and/or ceramic component and an osseointegrative and osteoinductive surface (multi)layer structure on the surface of the metallic and/or ceramic component, wherein the surface (multi)layer structure comprises at least one osseointegrative and osteoinductive layer wherein the osseointegrative and osteoinductive layer comprises strontium aluminate.

2. Implant of claim 1, wherein the content of the strontium aluminate is in the range of from 1 wt% to 50 wt%, preferably from 1 wt% to 20 wt% based on the total weight of the surface (multi)layer structure.

3. Implant according to one of the preceding claims, wherein the strontium aluminate is present in platelet-form.

4. Implant according to one of the preceding claims, wherein the osseointegrative and osteoinductive surface (multi)layer structure comprises several osseointegrative and osteoinductive layers.

5. Implant of claim 4, wherein the osseointegrative and osteoinductive layers of the surface (multi)layer structure have different strontium contents.

6. Implant of claim 5, wherein the strontium content increases with the layer number so that the outermost layer has the highest strontium content.

7. Implant according to one of the claims 5 or 6, wherein the amount of strontium aluminate in the innermost layer is >0 wt% and less than 5 wt%, preferably >0 wt% and ≤1 wt% based on the total weight of the surface (multi)layer structure.

8. Implant according to one of the preceding claims, wherein the surface (multi)layer structure comprises a ceramic base material, selected from the group consisting of alumina based ceramics and/or zirconia based ceramics,

9. Implant according to one of the preceding claims, wherein the ceramic base layer is selected from the group of zirconia-platelet toughened alumina ceramic.

10. Implant of claim 9, wherein the zirconia content in the zirconia platelet toughened alumina ceramic is up to 25 % based in the total weight of the surface (multi)layer structure.

11. Implant according to one of the preceding claims, wherein the surface of the ceramic component is structured.

12. Implant of claim 11, wherein the structuring of the surface is carried out by a process selected from the group consisting of a milling process, a turning process, a combination of a milling and a turning process, ceramic injection molding, a foaming process due to the addition of a foaming agent, a pore forming process during sintering due to the addition of a pore forming agent, a grinding process, electro discharge machining and additive manufacturing.

13. A method for producing an Implant in accordance with claims 1 to 12, wherein a ceramic slurry comprising strontium aluminate is applied to a substrate in green state.

14. Method for producing an Implant in accordance with claims 1 to 12, wherein the osseointegrative surface (multi)layer structure is applied to a substrate in sintered state.

15. Method of claim 14, wherein the osseointegrative surface (multi)layer structure is applied by vapour- or plasma deposition.

## Patentansprüche

1. Implantat, das eine Metall- und/oder eine Keramikkomponente und eine osseointegrative und osteoinduktive Oberflächen(mehrfach)schichtstruktur auf der Oberfläche der Metall- und/oder der Keramikkomponente umfasst, wobei die Oberflächen(mehrfach)schichtstruktur wenigstens eine osseointegrative und osteoinduktive Schicht umfasst, wobei die osseointegrative und osteoinduktive Schicht Strontiumaluminat umfasst.

2. Implantat nach Anspruch 1, wobei der Gehalt an Strontiumaluminat in dem Bereich von 1 Gew.-% bis 50 Gew.-%, vorzugsweise von 1 Gew.-% bis 20 Gew.-%, basierend auf dem Gesamtgewicht der Oberflächen(mehrfach)schichtstruktur, liegt.

3. Implantat nach einem der vorhergehenden Ansprüche, wobei das Strontiumaluminat in Plättchenform vorliegt.

4. Implantat nach einem der vorhergehenden Ansprüche, wobei die osseointegrative und osteoinduktive Oberflächen(mehrfach)schichtstruktur einige osseointegrative und osteoinduktive Schichten umfasst.

5. Implantat nach Anspruch 4, wobei die osseointegrativen und osteoinduktiven Schichten der Oberflächen(mehrfach)schichtstruktur unterschiedliche Strontiumgehalte aufweisen.

6. Implantat nach Anspruch 5, wobei der Strontiumgehalt mit der Schichtanzahl so zunimmt, dass die äußerste Schicht den höchsten Strontiumgehalt aufweist.

7. Implantat nach einem der Ansprüche 5 oder 6, wobei die Menge an Strontiumaluminat in der innersten Schicht >0 Gew.-% und weniger als 5 Gew.-%, vorzugsweise >0 Gew.-% und ≤1 Gew.-%, basierend auf dem Gesamtgewicht der Oberflächen(mehrfach)schichtstruktur beträgt.

8. Implantat nach einem der vorhergehenden Ansprüche, wobei die Oberflächen(mehrfach)schichtstruktur ein Keramikbasismaterial umfasst, das aus der Gruppe ausgewählt ist, die aus Keramik auf Aluminiumoxidbasis und/oder Keramik auf Zirkonoxidbasis besteht,

9. Implantat nach einem der vorhergehenden Ansprüche, wobei die Keramikbasisschicht aus der Gruppe von mit Zirkonoxidplättchen gehärteter Aluminiumoxidkeramik ausgewählt ist.

10. Implantat nach Anspruch 9, wobei der Zirkonoxidgehalt in der mit Zirkonoxidplättchen gehärteten Aluminiumoxidkeramik bis zu 25 % basierend auf dem Gesamtgewicht der Oberflächen(mehrfach)schichtstruktur beträgt.

11. Implantat nach einem der vorhergehenden Ansprüche, wobei die Oberfläche der Keramikkomponente strukturiert ist.

12. Implantat nach Anspruch 11, wobei die Strukturierung der Oberfläche durch einen Vorgang durchgeführt wird, der aus der Gruppe ausgewählt ist, die aus Folgendem besteht: einem Fräsvorgang, einem Drehvorgang, einer Kombination aus einem Fräs- und einem Drehvorgang, Keramikspritzgießen, einem Schäumvorgang infolge der Zugabe eines Schaummittels, einem Porenausbildungsvorgang während eines Sinterns infolge der Zugabe eines Porenausbildungsmittels, einem Schleifvorgang, funkenerosivem Abtragen und additivem Fertigen.

13. Verfahren zum Herstellen eines Implantats nach den Ansprüchen 1 bis 12, wobei ein Keramikschlicker, der Strontiumaluminat umfasst, auf ein Substrat in rohem Zustand aufgetragen wird.

14. Verfahren zum Herstellen des Implantats nach den Ansprüchen 1 bis 12, wobei die osseointegrative Oberflächen(mehrfach)schichtstruktur auf ein Substrat in gesintertem Zustand aufgetragen wird.

15. Verfahren nach Anspruch 14, wobei die osseointegrative Oberflächen(mehrfach)schichtstruktur durch Dampf- oder Plasmaabscheidung aufgetragen wird.

## Revendications

1. Implant comprenant un composant métallique et/ou céramique et une structure (multi)couche de surface ostéointégrative et ostéoinductive sur la surface du composant métallique et/ou céramique, la structure (multi)couche de surface comprenant au moins une couche ostéointégrative et ostéoinductive, la couche ostéointégrative et ostéoinductive comprenant de l'aluminate de strontium.

2. Implant selon la revendication 1, dans lequel la teneur en aluminate de strontium est comprise dans la plage de 1 à 50 % en poids, de préférence de 1 à 20 % en poids sur la base du poids total de la structure (multi)couche de surface.

3. Implant selon l'une des revendications précédentes, dans lequel l'aluminate de strontium est présent sous forme de plaquettes.

4. Implant selon l'une des revendications précédentes, dans lequel la structure (multi)couche de surface ostéointégrative et ostéoinductive comprend plusieurs couches ostéointégratives et ostéoinductives.

5. Implant selon la revendication 4, dans lequel les couches ostéointégratives et ostéoinductives de la structure (multi)couche de surface ont différentes teneurs en strontium.

6. Implant selon la revendication 5, dans lequel la teneur en strontium augmente avec le nombre de couches de telle sorte que la couche la plus externe a la teneur en strontium la plus élevée.

7. Implant selon l'une des revendications 5 ou 6, dans lequel la quantité d'aluminate de strontium dans la couche la plus interne est de > 0 % en poids et de moins de 5 % en poids, de préférence de > 0 % en poids et de ≤ 1 % en poids sur la base du poids total de la structure (multi)couche de surface.

8. Implant selon l'une des revendications précédentes, dans lequel la structure (multi)couche de surface comprend un matériau de base en céramique, choisi dans le groupe constitué par les céramiques à base d'alumine et/ou les céramiques à base de zircone,

9. Implant selon l'une des revendications précédentes, dans lequel la couche de base en céramique est choisie dans le groupe constitué par la céramique d'alumine renforcée par des plaquettes de zircone.

10. Implant selon la revendication 9, dans lequel la teneur en zircone dans la céramique d'alumine renforcée par des plaquettes de zircone est de 25 % maximum sur la base du poids total de la structure (multi)couche de surface.

11. Implant selon l'une des revendications précédentes, dans lequel la surface du composant en céramique est structurée.

12. Implant selon la revendication 11, dans lequel la structuration de la surface est réalisée par un processus choisi dans le groupe constitué par un processus de broyage, un processus de rotation, une combinaison d'un processus de broyage et d'un processus de rotation, un moulage par injection de céramique, un processus de moussage dû à l'ajout d'un agent moussant, un processus de formation de pores lors du frittage dû à l'ajout d'un agent de formation de pores, un processus de meulage, d'usinage par électroérosion et de fabrication d'additifs.

13. Procédé de production d'un implant selon les revendications 1 à 12, dans lequel une suspension de céramique comprenant de l'aluminate de strontium est appliquée sur un substrat à l'état vert.

14. Procédé de production d'un implant selon les revendications 1 à 12, dans lequel la structure (multi)couche de surface ostéointégrative est appliquée sur un substrat à l'état fritté.

15. Procédé selon la revendication 14, dans lequel la structure (multi)couche de surface ostéointégrative est appliquée par dépôt de vapeur ou de plasma.
